# FASCICULE DE BREVET EUROPEEN

(11) **EP 3 144 230 B1**
(45) Date de publication et mention de la délivrance du brevet: **11.09.2019**
(21) Numéro de dépôt: 16189512.3
(22) Date de dépôt: 19.09.2016
(51) Int. Cl.: B65B 7/28, A61J 1/06, A61J 1/20, A61M 5/30, A61M 19/00, B65B 7/16, B65B 65/06, B67B 1/04

(54) **PROCEDE D'OBTURATION D'UNE CARPULE COMPORTANT AU MOINS UN BOUCHON, MOYENS D'INSERTION ET LIGNE D'OBTURATION ASSOCIEE**
VERSCHLUSSVERFAHREN FÜR EINER KARPULE AUS MINDESTENS EINEM STOPFEN, MITTELN ZUM EINFÜHREN UND ZUGEHÖRIGER VERSCHLUSSLINIE
METHOD FOR OBTURATION OF A CARTRIDGE COMPRISING AT LEAST ONE PLUG, MEANS OF INSERTION AND ASSOCIATED OBTURATION LINE

(30) Priorité: 21.09.2015 FR 1558862
(43) Date de publication de la demande: 22.03.2017
(73) Titulaire: Disposable-Lab, 33650 Martillac (FR)
(72) Inventeur: ZAMBAUX, Jean Pascal, 33270 BOULIAC (FR); PAUCHDET, Bernard, 24150 SAINT CAPRAISE DE LALINDE (FR)
(74) Mandataire: Argyma

(56) Documents cités:
- WO-A1-2015/016170
- FR-A1- 2 957 592
- US-A- 3 737 973
- US-A- 5 519 984
- US-A1- 2007 175 538
- US-A1- 2015 122 693

## Description

La présente invention concerne un procédé d'obturation d'un contenant comportant au moins un bouchon, notamment une carpule.

L'invention couvre également les moyens d'insertion associés avec ses différentes variantes L'invention couvre enfin une ligne d'obturation de contenants, de façon industrielle.

Pour la suite de la description, le choix retenu pour exemplifier de façon non limitative est une application à une carpule pour le domaine médical.

La description s'appliquerait tout aussi bien à des contenants dont une extrémité est obturée et qui doivent être emplis et obturés à la seconde extrémité par un bouchon pénétrant dans ledit contenant, en générant un minimum d'air, comme un corps de seringue.

En effet, dans le domaine médical, les dispositifs d'injection intradermique à seringue sont fortement utilisés mais de nombreux patients peuvent présenter une phobie des aiguilles et de l'action engendrée par l'aiguille. C'est ainsi que les enfants peuvent y être sensibles mais les adultes aussi surtout lorsqu'il s'agit d'un traitement répétitif comme des traitements pour les allergies ou pour des prises quotidiennes dans le cas de traitements de longue durée.

Aussi, pour y remédier, il existe des moyens d'injection sans aiguille, notamment des moyens diffusés sous la marque Zenéo déposée la société CrossJect.

De tels moyens fonctionnent en propulsant le liquide contenu dans une carpule au moyen d'une mise sous très forte pression localisée de façon à faire pénétrer le liquide contenu dans ladite carpule à travers le derme. Un tête d'injection assure la diffusion du liquide en nombreux flux de très faibles diamètres.

Il n'y a donc plus d'aiguille et il suffit de positionner un applicateur sur le derme et de déclencher par simple pression la mise en oeuvre des moyens de mise sous très forte pression, ce qui provoque l'injection du liquide par projection à très grande vitesse à travers le derme.

On comprend que, si le liquide doit être mis sous très forte pression, de façon quasi instantanée, il faut que la carpule contenant ledit liquide soit exempte d'air ou du moins que le volume d'air soit aussi réduit que possible afin d'absorber le moins possible l'onde de pression exercée par les moyens de mise sous très forte pression.

Plus la transmission de la pression quasi instantanée au liquide sera intégrale et plus la pénétration sera efficace et de qualité.

Or le remplissage actuel peut être fortement amélioré.

Une carpule est constituée d'un contenant prenant la forme d'un tube, généralement en verre, de forte épaisseur pour résister à la pression, avec une lèvre périphérique aux extrémités pour son maintien et son positionnement.

Ce contenant, en forme de tube, est obturé à une première extrémité proximale par un premier bouchon, intégralement introduit dans le tube, par une extrémité. Ce bouchon est muni de moyens d'étanchéité avec la paroi dudit tube. Ce bouchon, ainsi positionné, reste néanmoins susceptible de se déplacer sous la forte pression qui sera exercée lors de son utilisation afin de libérer le liquide contenu dans ledit tube par cette extrémité proximale qui est équipée d'une tête de diffusion. Cette tête de diffusion a pour but de répartir le volume diffusé en de très nombreux jets de très faible diamètre.

Lors de la fabrication, le contenant en forme de tube, obturé par ce bouchon proximal, et destiné à porter une tête de diffusion, doit ensuite être empli du liquide à dispenser par son autre extrémité distale. Cette extrémité distale est ensuite obturée par un bouchon distal, en attendant son utilisation.

Le bouchon distal doit également pénétrer dans le tube intégralement d'une part du fait que les dimensions du contenant en forme de tube sont standards et que le volume de liquide à dispenser peut être variable et d'autre part, du fait que le second bouchon va recevoir et transmettre la très forte pression générée. Ce second bouchon va se comporter alors comme un piston pour transmettre cette très forte pression sous l'action des moyens de mise sous très forte pression.

De plus, les carpules sont utilisées comme des cartouches dans d'autres domaines comme le domaine dentaire, par exemple pour l'administration de produits d'anesthésie. Dans ce cas, cette administration recourt nécessairement à une seringue dans laquelle on introduit directement la carpule dite aussi cartouche dans ce milieu professionnel. Il faut, là aussi, limiter le volume d'air, pour transmettre la pression exercée sur le piston directement au fluide anesthésiant, sans un volume d'air tampon qui absorberait une partie de l'effort ou au moins avec un volume d'air minimum qui rendrait négligeable la capacité d'amortissement.

Les fluides anesthésiants présentent le plus souvent une viscosité importante nécessitant une forte pression du praticien pour assurer une bonne pénétration du produit anesthésiant dans la gencive.

Les carpules sont également utilisées dans le domaine vétérinaire ou le problème de remplissage des cartouches et autres carpules avec un minimum d'air se pose également.

Une solution connue pour emplir et obturer une carpule avec un bouchon qui consiste à boucher la carpule par son extrémité proximale, à emplir la carpule, à mettre la carpule sous vide, à introduire le bouchon distal lorsque la carpule est sous vide puis à casser le vide. Le bouchon distal se déplace alors dans le tube constituant la carpule.

Ce procédé présente un premier problème lié à la maîtrise du déplacement en translation du premier bouchon.

La reproductibilité est délicate et surtout dans le cas d'une erreur ou d'un déplacement incomplet, il n'y a aucune possibilité de "compléter" le déplacement ou de recommencer ou encore de réaspirer. Le contenu de la carpule est alors perdu, ce qui peut être très préjudiciable lorsque le produit est d'un coût élevé.

Généralement, le bouchon distal est constitué d'un corps de bouchon comportant au moins deux lèvres annulaires généralement trois, disposées l'une au-dessus de l'autre suivant l'axe longitudinal de la carpule et espacées les unes des autres.

Ces lèvres constituent l'étanchéité du bouchon distal et constituent autant de barrières à la fuite de la composition fluide contenue dans la carpule qu'à la pénétration de corps étrangers et de microorganismes à condition que lesdites lèvres restent exemptes de toute trace de composition liquide au droit de ces lèvres.

Le remplissage mécanique est donc très délicat et reste un problème à résoudre.

De plus, de telles carpules sont produites en grand nombre et il faut que le procédé comme le dispositif soient adaptés pour être mis en service sur des lignes de production industrielle à grande capacité et à forte cadence. La présente invention propose également une ligne d'obturation adaptée pour une application particulière à des carpules pour injection sans aiguille.

La reproductibilité est absolument essentielle afin de conférer à toutes les carpules produites les mêmes paramètres et de conduire à un usage avec des performances homogènes des moyens d'injection utilisant lesdites carpules.

Il est aussi nécessaire de proposer un procédé et des moyens d'introduction qui soient d'un coût non rédhibitoire.

Un document US 3 737 973 vise les corps de seringues pré-remplies et décrit un corps de seringue dans lequel est introduit un ensemble d'une tête de poussée comportant un pion central destiné à coopérer avec un logement borgne d'un bouchon destiné à venir obturer le corps de seringue une fois empli. La même tête est équipée d'une tige disposée radialement et latéralement par rapport à ladite tête et se prolongeant vers le bas, à la périphérie dudit bouchon.

La tête comporte en partie supérieure une tringle de manoeuvre en translation.

Ainsi le bouchon est emboîté sur le pion central de la tête et la tête munie de son bouchon et de sa tige radiale, latérale, est introduite, le tout simultanément, dans le corps de seringue et le bouchon est poussé, l'air s'échappant latéralement au bouchon durant la descente. Lorsque le bouchon est en place, la tête et sa tige radiale, latérale sont retirés et le bouchon reste en place, sans garantie qu'il ne bouge plus en translation puisque les éléments sont liés mécaniquement.

Le procédé et les moyens d'insertion selon la présente invention sont maintenant décrits en détail suivant un mode de réalisation particulier, non limitatif, cette description étant établie pour une application carpule sans pour autant être limitative à ce type de contenant. Cette description s'appuie sur le jeu de dessins annexés, dessins sur lesquels les différentes figures montrent, à titre illustratif uniquement :
- Figure 1 : une vue en perspective d'une carpule avec son premier bouchon supérieur, distal avant mise en place, et son second bouchon inférieur proximal en place,
- Figure 2 : une vue schématique des moyens d'insertion du premier bouchon,
- Figures 3A à 3F : une vue d'un synoptique du procédé d'obturation selon la présente invention, avec les différentes étapes,
- Figure 4 : une vue d'une variante du procédé avec une étape supplémentaire,
- Figures 5A à 5B : une vue de variantes de réalisation de la tige des moyens d'insertion,
- Figures 6A à 6H : une ligne de production avec les étapes associées pour l'obturation à l'aide du dispositif selon la présente invention.

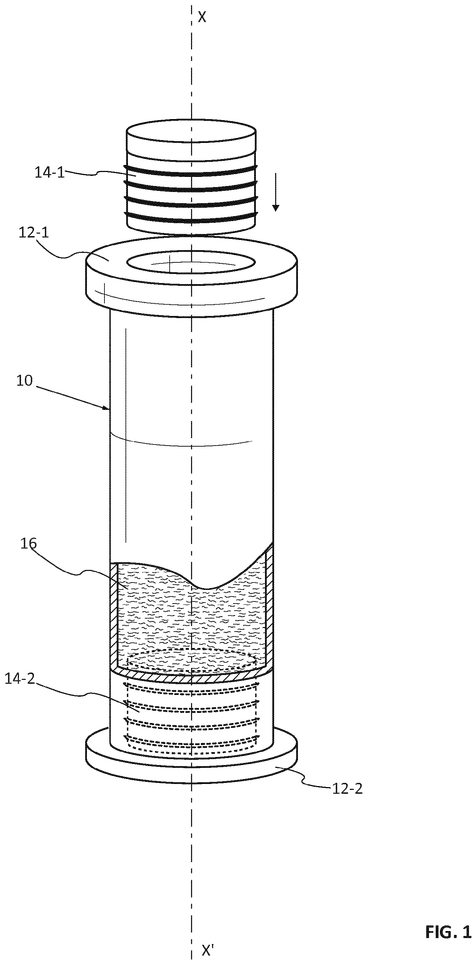

Sur la figure 1, on a représenté une carpule de type connu, servant d'illustration non limitative à la description qui suit. Cette carpule, de forme cylindrique comprend un corps 10 tubulaire, avec deux extrémités : une extrémité 10-1 supérieure et une extrémité 10-2 inférieure.

Cette carpule comporte deux couronnes supérieure 12-1 et inférieure 12-2. Ces couronnes 12 viennent se positionner à la périphérie extérieure des extrémités supérieure ou distale et inférieure ou proximale, du corps 10.

Chaque couronne est positionnée dans un plan perpendiculaire à l'axe longitudinal XX' de la carpule, passant par l'axe longitudinal du corps 10.

Le corps et les couronnes forment un ensemble monolithique et sont généralement en verre, ledit verre étant un verre épais de façon à pouvoir résister à une forte pression.

La carpule concernée est prévue pour recevoir deux bouchons, en un matériau du type élastomère : un premier bouchon 14-1 supérieur et un second bouchon 14-2 inférieur, libérateur.

Le second bouchon 14-2 inférieur est introduit dans une première opération afin d'obturer le corps 10 au droit de sa seconde extrémité 10-2, ce second bouchon étant un bouchon libérateur du produit sous l'action de la très haute pression.

Le corps 10 et le second bouchon 14-2 inférieur en place forment ainsi un contenant destiné à recevoir une composition 16 liquide. Le remplissage est effectué de façon connue, par exemple au moyen d'un nez doseur, non représenté.

Le premier bouchon 14-1 supérieur doit à son tour être introduit dans le corps 10 afin d'obturer l'extrémité supérieure du corps 10 tubulaire.

Le procédé d'obturation selon la présente invention propose les étapes suivantes pour parvenir à obturer avec le premier bouchon 14-1 en déplaçant ledit bouchon à proximité immédiate de la surface S libre de remplissage qui est la surface libre de la composition 16 liquide.

Le volume compris entre cette surface S libre de remplissage et le bouchon est dénommé "espace de tête" et doit être minimalisé pour résoudre les problèmes mentionnés en préambule.

Le procédé consiste à :
- Introduire au moins une partie de moyens 18 d'insertion parallèlement à l'intérieur du corps 10 du contenant, en l'occurrence la carpule,
- Insérer mécaniquement le premier bouchon 14-1 dans le corps 10, jusqu'à un niveau d'insertion dudit bouchon dans le corps tel que l'air de l'espace de tête est évacué entre le bouchon et les moyens 18 d'insertion, et
- Retirer au moins la partie des moyens 18 d'insertion en laissant le premier bouchon en place au niveau d'insertion de l'étape précédente.

Les moyens 18 d'insertion selon la présente invention comprennent notamment une tige 18-1, de section circulaire dans le mode de réalisation simplifié représenté.

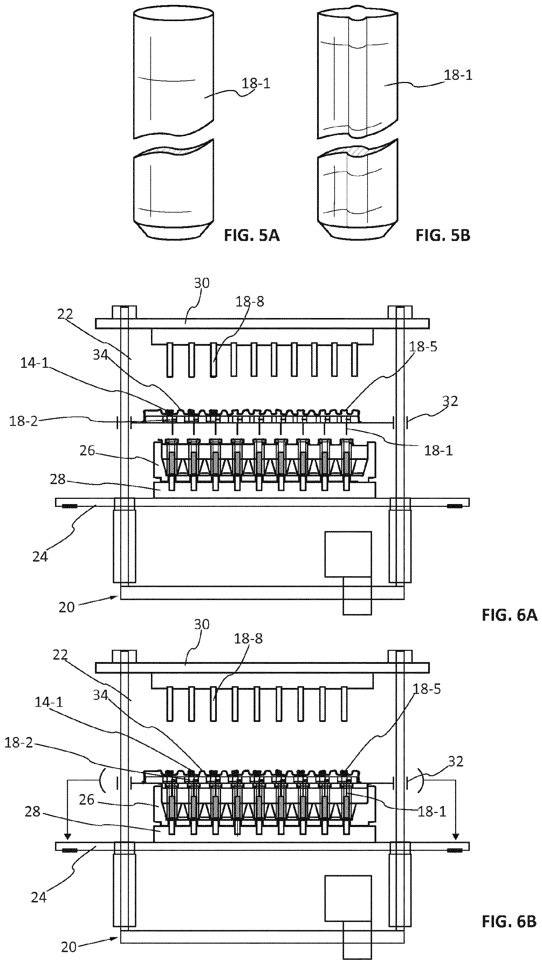

Cette tige 18-1 peut prendre différentes sections irrégulières, comme représentées sur les figures 5A et 5B, notamment une section elliptique et une section elliptique avec des bossages diamétraux.

Ces sections facilitent encore plus l'évacuation de l'air en générant des canaux C de sortie de section supérieure ou en générant, pour une même section de canaux, une résistance mécanique supérieure de la tige, comme explicité plus avant.

Lorsque le premier bouchon 14-1 est introduit mécaniquement, au moins une génératrice dudit bouchon, puisqu'il est cylindrique, vient en appui sur la tige 18-1 constituant une partie des moyens 18 d'insertion. Ceci génère donc au moins un canal C d'évacuation de l'air au fur et à mesure de la translation du bouchon dans le corps 10, suivant l'axe XX'.

La tige 18-1 est ensuite retirée du corps 10 de la carpule. Ceci est rendu possible car la surface de contact entre la tige 18-1 et le premier bouchon 14-1 supérieur génère des forces de frottement inférieures à celles qui sont générées entre la périphérie du bouchon et la paroi intérieure du corps 10 de la carpule.

Le premier bouchon 14-1, en matériau élastomère, reprend sa forme initiale et compense instantanément le retrait de la tige 18-1.

De façon préférentielle, selon une caractéristique du procédé, la tige 18-1 est positionnée avec son extrémité inférieure à proximité immédiate de la surface libre S de remplissage, en évitant tout contact avec la composition 16 liquide. En effet, un tel contact aurait pour conséquence de polluer l'espace entre la paroi intérieure du corps 10 et le premier bouchon 14-1 supérieur, lors du retrait de ladite tige 18-1.

Selon une autre caractéristique du présent procédé, il est possible d'adjoindre une étape supplémentaire qui consiste à incliner le corps 10 lors de son insertion. Cette inclinaison est effectuée suivant une génératrice, opposée à la génératrice recevant la tige, de façon que l'espace de tête se concentre du côté de la tige

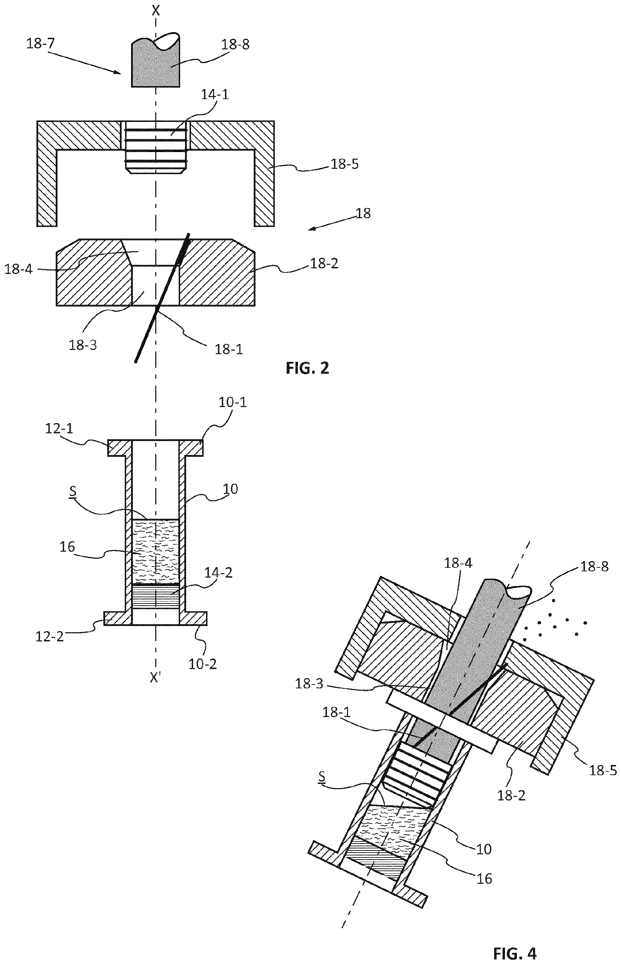

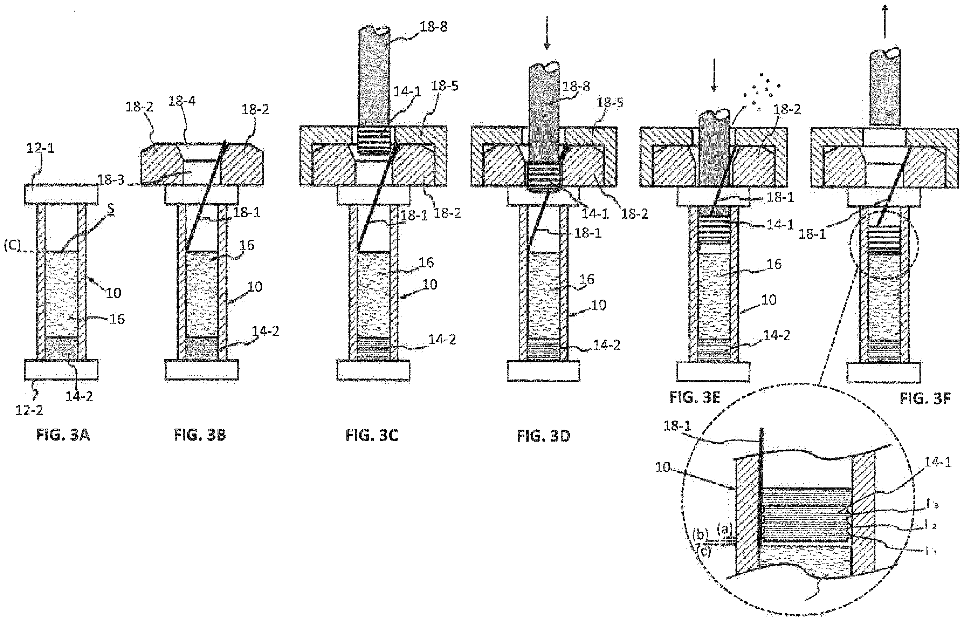

Des moyens 18 d'introduction du premier bouchon 14-1 supérieur sont maintenant décrits en détail en regard des figures 2 et 3, selon un mode de réalisation préférentiel.

Ces figures illustrent également le dispositif complet selon l'invention, permettant la mise en oeuvre du procédé qui vient décrit.

Sur la figure 2, les moyens 18 d'introduction comprennent une tige 18-1 solidaire d'une tête 18-2 de guidage qui comporte un passage 18-3 muni d'une entrée 18-4 en forme d'entonnoir. La tête 18-2 de guidage est de forme cylindrique et chanfreinée périphériquement, destinée à venir en appui et en référence sur l'extrémité 10-1 supérieure, et plus particulièrement sur la couronne 12-1 supérieure.

Les moyens 18 d'introduction comprennent de plus un porte bouchon 18-5 destiné à recevoir et à maintenir le premier bouchon 14-1 supérieur dans un logement traversant ménagé au centre dudit porte bouchon, et de façon coaxiale à l'axe XX'.

Le porte bouchon 18-5 est de profil conjugué de celui de la tête 18-2 de guidage de façon à venir coiffer ladite tête et à assurer un positionnement coaxial avant introduction.

Un actionneur 18-7 mécanique est schématiquement représenté sous la forme d'un piston 18-8.

La mise en oeuvre des moyens 18 d'insertion qui viennent d'être décrits est maintenant explicitée en regard des figures 3 qui représentent le synoptique correspondant.

Sur la figure 3A, on a représenté une carpule avec un corps 10 et deux couronnes supérieure 12-1 et inférieure 12-2. Ces couronnes 12 sont positionnées à la périphérie extérieure des extrémités 10-1 supérieure et 10-2 inférieure du corps 10.

Le second bouchon 14-2 inférieur est en place et obture l'extrémité 10-2 inférieure.

Le corps 10 a reçu un volume d'une composition 16 liquide dont le surface S libre de remplissage est à une hauteur (c).

La tête 18-2 de guidage est positionnée en appui et en référence sur l'extrémité supérieure 10-1 et sur la couronne 12-1.

Dans cette position, la tige 18-1 vient immédiatement au-dessus de la surface S libre de remplissage , sans contact, voir figure 3B.

La tête 18-2 de guidage peut aussi être coiffante sur la couronne pour un meilleur positionnement.

Sur la figure 3C, le porte bouchon 18-5 est rapporté de façon coaxiale sur la tête 18-2 de guidage.

Le passage 18-3 muni d'une entrée 18-4 en forme d'entonnoir est parfaitement centré sur l'axe XX'.

Le premier bouchon 14-1 supérieur est maintenu dans le logement traversant ménagé au centre dudit porte bouchon 18-5, et se trouve aussi positionné de façon coaxiale par rapport à l'axe XX'.

Le piston 18-8 est au-dessus du porte-bouchon 18-5, en attente et coaxial également avec l'axe XX'.

Sur la figure 3D, le piston 18-8 est mû en translation suivant l'indication de la flèche de cette figure.

Le piston 18-8 vient en appui sur la face supérieure du premier bouchon 14-1 supérieur et force son introduction dans l'entrée 18-4 en forme d'entonnoir, ce qui centre encore ledit bouchon et provoque une certaine compression radiale dudit bouchon.

Lors de la translation, le bord périphérique du bouchon entre en contact avec la tige 18-1, ce qui génère au moins un canal Ç qui autorise l'évacuation de l'air, au fur et à mesure de l'insertion par translation du premier bouchon14-1 supérieur dans le corps 10. Le bouchon quitte le porte-bouchon 18-5 qui n'a plus alors de fonction.

Sur la figure 3E, on constate que le premier bouchon 14-1 descend et que l'air s'échappe.

Le piston a poussé le bouchon 14-1 jusqu'à une position référencée (b) en laissant un espace de tête très réduit.

Le bouchon 14-1 est généralement constitué de matière élastomère et comprend en étanchéité au moins deux lèvres, en l'occurrence trois lèvres l1, l2 et l3, l1 étant la plus proche de l'extrémité inférieure, plus la partie supérieure du corps du bouchon. Les dimensions sont adaptées en fonction des besoins, de la nature des matériaux, l'intérieur du corps 10 pouvant subir préalablement un traitement de surface adapté.

La composition 16 liquide voit sa surface S libre de remplissage dans une position c et la première lèvre l1 est dans une position (a).

On constate que la position de la tige doit être située, au plus bas, immédiatement au-dessus de surface libre S de remplissage du liquide contenu dans le corps du contenant, au moins au-dessus de la position (c).

Ceci évite que de la composition 16 liquide ne vienne au contact d l'extrémité de la tige 18-1 et la pollue.

La première lèvre l1 assure donc bien une première barrière efficace, sans trace de composition 16 liquide immiscée entre ladite lèvre et la paroi intérieure du corps 10.

Sur cette figure 3F, le piston 18-8 est retiré et le bouchon 14-1 reste en place. On note que les autres lèvres l2 et l3 reprennent également leur position d'étanchéité du fait de la nature élastomérique dudit matériau dès le retrait de la tige.

De même, le frottement sur ce matériau élastomère reste totalement négligeable et il ne provoque aucun mouvement du bouchon lors du retrait de la tige.

Concernant la tige, pour ne pas endommager d'une quelconque façon la face intérieure du corps tubulaire, pour faciliter la mise en place du premier bouchon, il est possible de prévoir un traitement de surface de ladite paroi.

Une solution avantageuse consiste aussi à réaliser ladite tige à partir d'un matériau connu sous le nom de PEEK qui est le polyéthercétone, présentant des propriétés mécaniques largement suffisantes pour l'application de la présente invention et dont le coefficient de frottement est très limité.

Il suffit ensuite de retirer le porte bouchon 18-5 qui est exempt de bouchon puis la tête 18-2 de guidage.

Ces deux retraits peuvent être effectués simultanément.

Selon un perfectionnement de la présente invention, il est prévu de pouvoir légèrement incliner le corps 10 comme montré sur la figure 4 de façon à concentrer le volume d'air en fin de poussée sur le bouchon en un endroit se situant au droit de l'extrémité de la tige 14-1.

Ainsi l'espace de tête est concentré en un point.

Cette étape est facultative car, en fonction des produits, la mouillabilité de la composition 16 liquide est suffisante pour venir immédiatement sous la face inférieure du premier bouchon 14-1. Le volume de tête est alors réduit à la couronne périphérique située sous la première lèvre l1, ce qui est extrêmement limité et totalement acceptable notamment pour l'application envisagée d'administration à partir d'une carpule sous très haute pression.

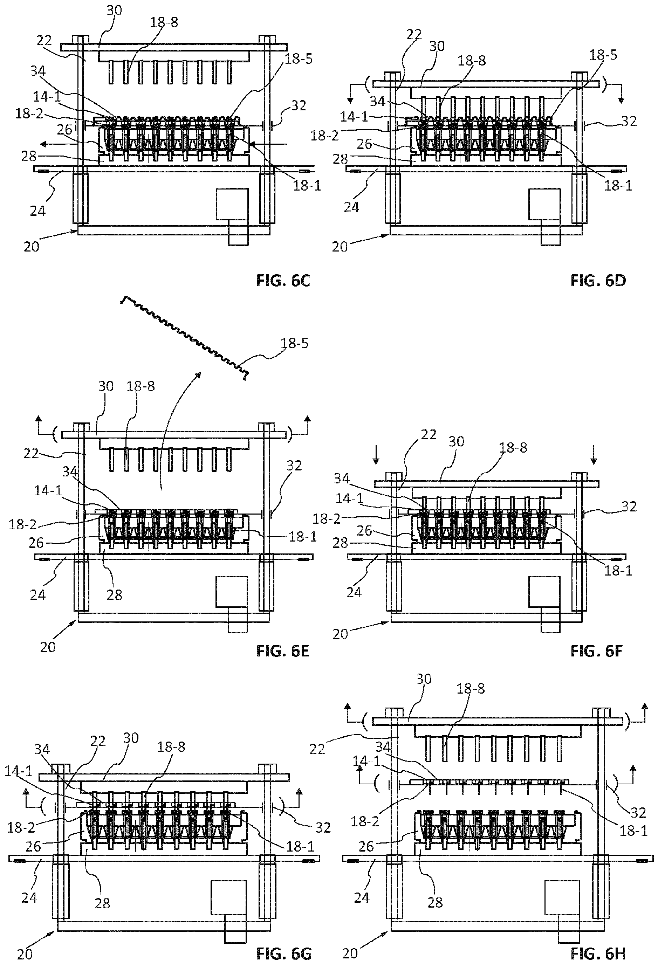

Ceci est d'autant plus acceptable pour les seringues unidose pré remplies à pression manuelle. Concernant le procédé et les moyens d'obturation permettant sa mise en oeuvre, on constate qu'il est possible d'industrialiser ce procédé et une illustration d'une ligne de production industrielle est maintenant décrite en regard des figures 6.

Les repères dans la ligne de production industrielle restent identiques.

Dans le cas présent, la ligne comprend 72 poses simultanées.

Sur la figure 6A, on a représenté schématiquement une presse de mise en place qui comporte un bâti 20 avec des colonnes 22 de guidage, une platine 24 inférieure destinée à recevoir un plateau 26 porte-contenants, en l'occurrence des carpules si l'on poursuit la description pour cette application particulière aux carpules. Cette platine 24 inférieure comprend des moyens 28 permettant de déplacer le plateau 26 porte-carpules en translation horizontale.

Il est aussi prévu une platine 30 supérieure qui est mobile en translation sur les colonnes 22 de guidage. Cette platine 30 supérieure porte un ensemble de pistons 18-8 avec la même répartition géométrique que celle des carpules.

Une troisième platine 32 intermédiaire est également mobile en translation sur les colonnes 22 de guidage, parallèlement à la platine 30 supérieure. Cette platine 32 intermédiaire comprend un plateau 34 avec autant de porte bouchons 18-2 et de tiges 18-1 que de carpules avec la même répartition géométrique. Les tiges 18-1 sont décentrées par rapport aux bouchons 14-1 de façon à être positionnées sensiblement au droit de la périphérie de chaque bouchon.

Le bâti 20 peut aussi, bien que cela ne soit pas représenté, comporter une zone "sale" et une zone "propre" voire stérile afin de conserver les carpules dans la zone propre.

Le bâti étant 20 étant dans la configuration de la figure 6A, la platine 24 inférieure porte un plateau 26 porte-carpules, lesdites carpules étant emplies d'une composition 16 liquide et ayant reçues préalablement un second bouchon d'obturation de l'extrémité inférieure 10-2.

Ces étapes ne font pas partie de l'invention car parfaitement connues en elles-mêmes.

La platine 32 intermédiaire est positionnée au-dessus de la platine 24 inférieure pour permettre la dépose du plateau 26 porte-carpules. Les tiges 18-1 sont alors centrées par rapport à l'axe XX' des carpules et les bouchons 14-1 dans le porte-bouchons sont décentrés.

Sur la figure 6B, la première étape est la descente de la platine 32 intermédiaire qui assure une mise en place des tiges 18-1 de façon centrée et donc coaxiale avec l'axe XX' des carpules.

L'étape de la figure 6C consiste à provoquer un déplacement en translation horizontal jusqu'à ce que les tiges viennent le long d'une génératrice intérieure du corps 10 de chaque carpule.

Les bouchons 14-1 sont alors centrés par rapport aux carpules et les têtes 18-2 de guidage également. Les tiges 18-1 sont, elles, décentrées, positionnées le long d'une génératrice intérieure du corps 10.

Cette étape est facultative mais constitue néanmoins le meilleur mode de réalisation car elle évite l'appui accidentel d'au moins une tige 18-1 sur le partie annulaire 12-1 supérieure d'une carpule, ce qui pourrait provoquer la rupture et pour le moins la déformation de la tige concernée.

A l'étape 6D, la platine 30 supérieure est déplacée en translation vers le bas, ce qui provoque la descente simultanée des pistons 18-8.

Les bouchons 14-1 sont donc extraits du plateau 34 porte-bouchon et pénètrent à travers chaque tête 18-2 de guidage respective, dans le passage 18-4 en entonnoir puis dans le passage 18-3.

La platine 30 supérieure avec ses pistons est ensuite ramenée en position initiale par une translation vers le haut, comme montré sur la figure 6E, afin de permettre le retrait du plateau 34 porte-bouchons.

Sur la figure 6F, la platine 30 supérieure est descendue de nouveau mais plus bas pour pousser les bouchons 14-1 hors de la tête de guidage, dans le corps 10, et pour diminuer l'espace de tête. Chaque bouchon est déplacé en translation de la tête de guidage qui le maintenait dans le corps de la carpule correspondante.

Chaque bouchon étant poussé en translation le long de la paroi intérieure du corps 10 de chaque carpule et le long de la tige 18-1 associée, l'air ou le gaz neutre en cas d'obturation sous atmosphère neutre, peut s'échapper à travers les canaux Ç ainsi formés, comme expliqué plus haut dans la description.

Le niveau de descente est réglé en fonction des données de remplissage pour obtenir, pour chaque carpule, un remplissage de la composition liquide à un niveau (c), une extrémité inférieure du bouchon 14-1 à un niveau (b) et une extrémité de la tige à un niveau (a).

Il est bien entendu qu'il est possible de prévoir la mise en place de plusieurs tiges pour chaque carpule de façon à générer plus de canaux d'évacuation de l'air ou du gaz et/ou de symétriser l'opération d'obturation. Ceci peut permettre un meilleur équilibrage du bouchon durant la descente.

La figure 6G prévoit le déplacement en translation de la platine 32 intermédiaire en laissant en place les pistons 18-8 et donc la platine 30 supérieure, en position basse. Ceci évite tout déplacement de chaque bouchon lors du retrait des aiguilles. Le risque est faible mais le maintien des bouchons pendant le retrait des aiguilles est une garantie supplémentaire.

La longueur des pistons doit être prévue en conséquence.

La figure 6H montre le retour de la platine 30 en position haute.

Le plateau de carpules emplies et obturées avec un espace de tête, minimalisé, est prêt pour être préparé pour la commercialisation. Les carpules du plateau peuvent être intégrés à un applicateur, notamment à des carters de dispositifs d'injection sans aiguille.

Selon une variante du procédé, la tige 18-1 et le bouchon 14-1 peuvent être introduits simultanément.

Le procédé d'obturation permet ainsi de minimaliser l'espace de tête, les moyens d'obturation sont simples et industriels et la ligne de production répond aux besoins industriels.

## Revendications

1. Procédé d'obturation d'une carpule contenant un liquide, ladite carpule comprenant un corps (10) avec une ouverture à au moins une extrémité et comportant au moins un bouchon (14-1) d'obturation, ledit bouchon comprenant au moins deux lèvres annulaires (l1-l3), destiné à venir obturer ladite ouverture après remplissage, ledit remplissage définissant une surface S libre de remplissage, le remplissage ayant laissé subsister un espace de tête dans ladite carpule, **caractérisé en ce qu'**il consiste à réaliser, successivement, les différentes étapes suivantes :
a) Introduction d'au moins une tige (18-1) parallèlement à une paroi intérieure du corps (10) de ladite carpule, jusqu'à ce que son extrémité soit immédiatement au-dessus de surface libre S de remplissage de la composition (16) liquide, contenue dans le corps (10) de la carpule,
b) Insertion mécanique dudit bouchon (14-1) dans le corps (10), le long de la au moins une tige (18-1) d'insertion, générant au moins un canal C, l'air de l'espace de tête étant évacué entre ledit bouchon et ladite tige,
c) Retrait de ladite au moins une tige (18-1) en laissant ledit bouchon en place au niveau d'insertion de l'étape précédente.

2. Procédé d'obturation d'une carpule avec un corps (10) comportant une ouverture à au moins une extrémité, selon la revendication 1, **caractérisé en ce que** la tige (18-1) d'insertion est introduite pour que son extrémité soit positionnée au droit de la lèvre (l1) la plus proche de la surface S libre de la composition (16) liquide.

3. Procédé d'obturation d'une carpule avec un corps comportant une ouverture à au moins une extrémité, selon la revendication 1 ou 2, **caractérisé en ce que**, dans le cas d'un corps (10) cylindrique, la direction d'introduction de la tige (18-1) est parallèle à une génératrice.

4. Procédé d'obturation d'une carpule avec un corps (10) comportant une ouverture à au moins une extrémité, selon l'une quelconque des revendications précédentes, **caractérisé en ce que** le corps (10) et/ou la tige (18-1) et/ou le bouchon (14-1) subissent un traitement de surface préalable ou sont en un matériau à faible coefficient de frottement, pour diminuer le coefficient de friction.

5. Procédé d'obturation d'une carpule avec un corps (10) comportant une ouverture à au moins une extrémité, selon l'une quelconque des revendications précédentes, **caractérisé en ce que** le bouchon (14-1) est maintenu en place mécaniquement durant l'étape c/ de retrait de la tige (18-1).

6. Procédé d'obturation d'une carpule avec un corps (10) comportant une ouverture à au moins une extrémité, selon l'une quelconque des revendications précédentes, **caractérisé en ce que** le corps (10) du contenant est incliné lors de l'évacuation de l'air en positionnant le contenant de façon que l'extrémité de la tige (18-1) vienne en point haut de l'espace de tête.

7. Procédé d'obturation d'une carpule-avec un corps (10) comportant une ouverture à au moins une extrémité, selon l'une quelconque des revendications précédentes, **caractérisé en ce que** le corps (10) est une carpule comprenant, en plus de l'extrémité supérieure (10-1) obturée par le bouchon (14-1) d'obturation, une autre extrémité (10-2) préalablement close avec un bouchon (14-2) libérateur.

8. Dispositif d'obturation d'une carpule avec un corps (10) comportant une ouverture à au moins une extrémité, pour la mise en oeuvre du procédé selon l'une quelconque des revendications précédentes, **caractérisé en ce qu'**il comprend une tête (18-2) de guidage d'un bouchon (14-1) dans ladite carpule emplie, au moins une tige (18-1) et un porte-bouchon (18-5) ainsi qu'un actionneur (18-7) mécanique comprenant au moins un piston (18-8).

9. Dispositif d'obturation d'une carpule avec un corps (10) comportant une ouverture à au moins une extrémité, selon la revendication 8, **caractérisé en ce que** la tige (18-1) a une section en forme de disque.

10. Dispositif d'obturation d'une carpule avec un corps (10) comportant une ouverture à au moins une extrémité, selon la revendication 8, **caractérisé en ce que** la tige (18-1) a une section irrégulière.

11. Ligne de production de carpules par mise en oeuvre du procédé selon l'une des revendications 1 à 7 et du dispositif selon la revendication 8, 9 ou 10, chaque carpule comprenant un corps (10) avec une première et une seconde extrémité (10-1, 10-2) ouvertes, **caractérisée en ce qu'**elle comprend une presse de mise en place comprenant :
- un bâti (20) avec des colonnes (22) de guidage,
- une platine (24) inférieure destinée à recevoir un plateau (26) porte-carpules, avec une géométrie de répartition,
- une platine (30) supérieure, mobile en translation sur les colonnes (22), munie d'un ensemble de pistons (18-8), avec la même géométrie de répartition que les carpules,
- une platine (32) intermédiaire également mobile en translation sur les colonnes (22) de guidage, parallèlement à ladite platine (30) supérieure, munie d'un plateau (34) porte bouchons (18-2) avec autant de bouchons (14-1) et de tiges (18-1) que de carpules avec la même géométrie de répartition,

12. Ligne de production de carpules selon la revendication 11, **caractérisée en ce que** la platine (24) inférieure comprend des moyens (28) permettant de déplacer le plateau (26) porte-carpules en translation horizontale.

13. Ligne de production de carpules selon la revendication 11 ou 12, **caractérisée en ce que** les tiges (18-1) sont décentrées par rapport aux bouchons (14-1) de façon à être positionnées sensiblement au droit de la périphérie de chaque bouchon (14-1).

## Patentansprüche

1. Verschlussverfahren für eine Karpule, die eine Flüssigkeit enthält, wobei die Karpule einen Körper (10) mit einer Öffnung an mindestens einem Ende umfasst und mindestens einen Verschlussstopfen (14-1) aufweist, wobei der Stopfen mindestens zwei ringförmige Lippen (l1-l3) umfasst, die zum Verschließen der Öffnung nach dem Füllen bestimmt sind, wobei das Füllen eine freie Fülloberfläche S bestimmt, wobei beim Füllen ein Kopfraum in der Karpule verbleibt,
**dadurch gekennzeichnet, dass** es darin besteht, schrittweise die folgenden unterschiedlichen Schritte durchzuführen:
a) Einführen mindestens eines Stabs (18-1) parallel zu einer Innenwand des Körpers (10) der Karpule, bis sich sein Ende unmittelbar über der freien Fülloberfläche S der Flüssigkeitszusammensetzung (16) befindet, die in dem Körper (10) der Karpule enthalten ist,
b) mechanisches Einsetzen des Stopfens (14-1) in den Körper (10) entlang des mindestens einen Einsetzstabs (18-1), wodurch mindestens ein Kanal C geschaffen wird, wobei die Luft des Kopfbereichs zwischen dem Stopfen und dem Stab ausgeleitet wird,
c) Entfernen des mindestens einen Stabs (18-1), wobei der Stopfen im Einsetzbereich des vorangehenden Schritts am Platz verbleibt.

2. Verschlussverfahren für eine Karpule mit einem Körper (10), der an mindestens einem Ende eine Öffnung aufweist, nach Anspruch 1, **dadurch gekennzeichnet, dass** der Einsetzstab (18-1) eingeführt wird, damit sein Ende senkrecht zu der Lippe (l1) positioniert ist, welche der freien Oberfläche S der Flüssigkeitszusammensetzung (16) am nächsten ist.

3. Verschlussverfahren für eine Karpule mit einem Körper, der an mindestens einem Ende eine Öffnung aufweist, nach Anspruch 1 oder 2, **dadurch gekennzeichnet, dass** im Fall eines zylindrischen Körpers (10) die Einführrichtung des Stabs (18-1) parallel zu einer Mantellinie ist.

4. Verschlussverfahren für eine Karpule mit einem Körper (10), der an mindestens einem Ende eine Öffnung aufweist, nach einem der vorangehenden Ansprüche, **dadurch gekennzeichnet, dass** der Körper (10) und/oder der Stab (18-1) und/oder der Stopfen (14-1) eine vorherige Oberflächenbearbeitung erfahren oder aus einem Material mit einem geringen Reibungskoeffizienten sind, um den Gleitreibungskoeffizienten zu verringern.

5. Verschlussverfahren für eine Karpule mit einem Körper (10), der an mindestens einem Ende eine Öffnung aufweist, nach einem der vorangehenden Ansprüche, **dadurch gekennzeichnet, dass** der Stopfen (14-1) während des Schritts c/ des Entfernens des Stabs (18-1) mechanisch am Platz gehalten wird.

6. Verschlussverfahren für eine Karpule mit einem Körper (10), der an mindestens einem Ende eine Öffnung aufweist, nach einem der vorangehenden Ansprüche, **dadurch gekennzeichnet, dass** der Körper (10) des Behälters beim Ausleiten der Luft durch Positionieren des Behälters derart geneigt wird, dass das Ende des Stabs (18-1) an den oberen Punkt des Kopfbereichs gelangt.

7. Verschlussverfahren für eine Karpule mit einem Körper (10), der an mindestens einem Ende eine Öffnung aufweist, nach einem der vorangehenden Ansprüche, **dadurch gekennzeichnet, dass** der Körper (10) eine Karpule ist, die zusätzlich zu dem oberen Ende (10-1), das mit dem Verschlussstopfen (14-1) verschlossen ist, ein anderes Ende (10-2) umfasst, das zuvor mit einem Freigabestopfen (14-2) geschlossen wird.

8. Verschlussvorrichtung für eine Karpule mit einem Körper (10), der an mindestens einem Ende eine Öffnung aufweist, für die Durchführung des Verfahrens nach einem der vorangehenden Ansprüche, **dadurch gekennzeichnet, dass** sie einen Führungskopf (18-2) eines Stopfens (14-1) in die gefüllten Karpule, mindestens einen Stab (18-1) und einen Stopfenträger (18-5) sowie einen mechanischen Aktuator (18-7) umfasst, der mindestens einen Kolben (18-8) umfasst.

9. Verschlussvorrichtung für eine Karpule mit einem Körper (10), der an mindestens einem Ende eine Öffnung aufweist, nach Anspruch 8, **dadurch gekennzeichnet, dass** der Stab (18-1) einen Querschnitt in Scheibenform hat.

10. Verschlussvorrichtung für eine Karpule mit einem Körper (10), der an mindestens einem Ende eine Öffnung aufweist, nach Anspruch 8, **dadurch gekennzeichnet, dass** der Stab (18-1) einen unregelmäßigen Querschnitt hat.

11. Produktionslinie von Karpulen mittels Durchführung des Verfahrens nach einem der Ansprüche 1 bis 7 und der Vorrichtung nach Anspruch 8, 9 oder 10, wobei jede Karpule einen Körper (10) mit einem ersten und einem zweiten offenen Ende (10-1, 10-2) umfasst, **dadurch gekennzeichnet, dass** sie eine Platzierungspresse umfasst, umfassend:
- ein Gestell (20) mit Führungssäulen (22),
- eine untere Platte (24), die zur Aufnahme eines Karpulenträgertabletts (26) mit einer Verteilungsgeometrie bestimmt ist,
- eine auf den Säulen (22) translatorisch bewegliche obere Platte (30), die mit einer Kolbeneinheit (18-8) mit derselben Verteilungsgeometrie wie die Karpulen ausgestattet ist,
- eine zu der oberen Platte (30) parallele, ebenfalls auf den Führungssäulen (22) translatorisch bewegliche Zwischenplatte (32), die mit einem Tablett (34) für Stopfenträger (18-2) mit ebenso vielen Stopfen (14-1) und Stäben (18-1) wie Karpulen mit derselben Verteilungsgeometrie ausgestattet ist.

12. Produktionslinie von Karpulen nach Anspruch 11, **dadurch gekennzeichnet, dass** die untere Platte (24) Mittel (28) umfasst, die erlauben, das Karpulenträgertablett (26) translatorisch horizontal zu verlagern.

13. Produktionslinie von Karpulen nach Anspruch 11 oder 12, **dadurch gekennzeichnet, dass** die Stäbe (18-1) in Bezug auf die Stopfen (14-1) derart dezentriert sind, dass sie etwa senkrecht zur Peripherie jedes Stopfens (14-1) positioniert sind.

## Claims

1. A method for sealing a carpule containing a liquid, said carpule comprising a body (10) with an aperture at at least one end and including at least one sealing plug (14-1), said plug comprising at least two annular lips (11-13), for sealing said aperture after filling, said filling defining a free filling area S, the filling having left a head space in said carpule,
**characterised in that** it consists in performing, successively, the different following steps of:
a) introducing at least one rod (18-1) in parallel to an internal wall of the body (10) of said carpule, until its end is immediately above the free filling area S of the liquid composition (16), contained in the body (10) of the carpule,
b) mechanically inserting said plug (14-1) in the body (10), along the at least one insertion rod (18-1), generating at least one channel C, the air of the head space being discharged between said plug and said rod,
c) removing said at least one rod (18-1) by leaving said plug in place at the insertion level of the previous step.

2. The method for sealing a carpule with a body (10) including an aperture at at least one end according to claim 1, **characterised in that** the insertion rod (18-1) is introduced such that its end is positioned flush with the lip (11) closest to the free area S of the liquid composition (16).

3. The method for sealing a carpule with a body including an aperture at at least one end according to claim 1 or 2, **characterised in that**, in the case of a cylindrical body (10), the direction of introduction of the rod (18-1) is parallel to a generatrix.

4. The method for sealing a carpule with a body (10) including an aperture at at least one end according to any of the preceding claims, **characterised in that** the body (10) and/or the rod (18-1) and/or the plug (14-1) undergo a prior surface treatment or are of a low friction material, to decrease the friction coefficient.

5. The method for sealing a carpule with a body (10) including an aperture at at least one end according to any of the preceding claims, **characterised in that** the plug (14-1) is mechanically held in place during step c/ of removing the rod (18-1).

6. The method for sealing a carpule with a body (10) including an aperture at at least one end according to any of the preceding claims, **characterised in that** the body (10) of the container is tilted upon discharging air by positioning the container such that the end of the rod (18-1) arrives at the topmost point of the head space.

7. The method for sealing a carpule with a body (10) including an aperture at at least one end according to any of the preceding claims, **characterised in that** the body (10) is a carpule comprising, in addition to the upper end (10-1) sealed by the sealing plug (14-1), another end (10-2) previously closed with a releasing plug (14-2).

8. A device for sealing a carpule with a body (10) including an aperture at at least one end, for implementing the method according to any of the preceding claims, **characterised in that** it comprises a head (18-2) for guiding a plug (14-1) into said carpule filled, at least one rod (18-1) and a plug carrier (18-5) as well as a mechanical actuator (18-7) comprising at least one piston (18-8).

9. The device for sealing a carpule with a body (10) including an aperture at at least one end according to claim 8, **characterised in that** the rod (18-1) has a disc shaped cross-section.

10. The device for sealing a carpule with a body (10) including an aperture at at least one end according to claim 8, **characterised in that** the rod (18-1) has an irregular cross-section.

11. A production line for carpules by implementing the method according to one of claims 1 to 7 and the device according to claim 8, 9 or 10, each carpule comprising a body (10) with a first and a second open end (10-1, 10-2), **characterised in that** it comprises a placement press comprising:
- a framework (20) with guiding columns (22),
- a lower plate (24) for receiving a carpule carrier tray (26), with a distribution geometry,
- an upper plate (30) translationally movable on the columns (22), provided with a set of pistons (18-8), with the same distribution geometry as the carpules,
- an intermediate plate (32) also translationally movable on the guiding columns (22), in parallel to the upper plate (30), provided with a plug carrier tray (18-2) with as many plugs (14-1) and rods (18-1) as carpules with the same distribution geometry.

12. The carpule production line according to claim 11, **characterised in that** the lower plate (24) comprises means (28) for moving the carpule carrier tray (26) in a horizontal translation.

13. The carpule production line according to claim 11 or 12, **characterised in that** the rods (18-1) are off-centred with respect to the plugs (14-1) so as to be substantially positioned flush with the periphery of each plug (14-1).
